# EUROPÄISCHE PATENTSCHRIFT

(11) **EP 2 590 427 B1**
(45) Veröffentlichungstag und Bekanntmachung des Hinweises auf die Patenterteilung: **02.08.2017**
(21) Anmeldenummer: 12190323.1
(22) Anmeldetag: 29.10.2012
(51) Int. Cl.: A61B 90/30, H04N 5/225, H04N 21/60

(54) **Handgriff**
Handle
Poignée

(30) Priorität: 04.11.2011 DE 102011117700
(43) Veröffentlichungstag der Anmeldung: 08.05.2013
(73) Patentinhaber: Berchtold GmbH & Co. KG, 78532 Tuttlingen (DE)
(72) Erfinder: Tockweiler, Udo, 78194 Immendingen (DE)
(74) Vertreter: Manitz Finsterwald Patentanwälte PartmbB

(56) Entgegenhaltungen:
- DE-A1-102009 051 994
- US-A1- 2011 122 260
- US-B1- 6 633 328

## Beschreibung

Die vorliegende Erfindung betrifft einen Handgriff für eine Operationsleuchte nach dem Oberbegriff des Anspruchs 1, umfassend ein Gehäuse, das zumindest bereichsweise nicht geschirmt ist und eine HD-Kamera, die im Inneren des Handgriffs angeordnet ist.

Derartige Handgriffe sind grundsätzlich bekannt, wobei die Bildsignale der HD-Kamera über Stecker an die Operationsleuchte übertragen werden, in dieser durch eine Komprimierungseinrichtung in Echtzeit komprimiert werden und dann über eine Übertragungsleitung von der Operationsleuchte zu einer externen Dekomprimierungseinrichtung übermittelt werden.

Da Operationsleuchten an Gestängen aufgehängt werden, die über um 360° drehbare Gelenke verfügen, ist es erforderlich, die Übertragungsleitung an den Gelenken durch 360°-Drehstecker und 360°-Schleifkontakte zu ersetzen, um ein Verschwenken der Aufhängung zu ermöglichen und eine Beschädigung der Übertragungsleitung zu verhindern.

Die zu verwendenden speziellen Schleifkontakte und Stecker müssen dabei hohen Anforderungen, z.B. in Bezug auf die elektromagnetische Schirmung zur Verringerung der EMV-Belastung oder die zu übermittelnden Datenraten, genügen. Die Übertragung des HD-Videosignals vom Handgriff zu der Dekomprimierungseinrichtung ist somit sehr aufwändig.

Es ist das der Erfindung zugrunde liegende Problem, einen Handgriff für Operationsleuchten der eingangs genannten Art dahingehend weiterzubilden, dass eine möglichst einfache Übertragung der HD-Videosignale an eine Dekomprimierungseinrichtung ermöglicht wird, wobei gleichzeitig die Verschwenkbarkeit der Operationsleuchtenaufhängung erhalten bleibt.

Die Lösung dieser Aufgabe erfolgt durch die Merkmale des Anspruchs 1 und insbesondere dadurch, dass der Handgriff im Inneren eine Bildverarbeitungseinrichtung, eine Komprimierungseinrichtung und eine Funkübertragungseinrichtung besitzt, wobei zumindest eine Antenne vollständig im Inneren des Handgriffs angeordnet ist, die zur Übermittlung eines komprimierten HD-Videosignals dient.

Mit der erfindungsgemäßen Lösung kann das von der HD-Videokamera erzeugte Videosignal innerhalb des Handgriffs in Echtzeit komprimiert werden. Durch z.B. eine H.264 Komprimierung von Full HD-Videoclips wird die hohe Datenrate der Videoclips auf ein Maß verringert, das deren Übertragung durch eine Funkübertragungseinrichtung erlaubt. Somit ergibt sich eine deutliche Vereinfachung des Aufbaus der Halterungsgestänge von Operationsleuchten, da in diesen Gestängen nun keine Signalleitungen bzw. Übertragungsleitungen, Spezialstecker oder 360°-Schleifkontakte für die Übertragung des HD-Videosignals untergebracht werden müssen. Der Handgriff ist somit unabhängig von den verwendeten Signalübertragungsleitungen in jeder Operationsleuchte einsetzbar, die über eine geeignete Stromversorgung verfügt. Außerdem kann auf einfache Weise, nämlich nur durch Auswechseln des Handgriffs, eine Umrüstung von SD auf HD-Bildverarbeitung erfolgen.

Durch die vollständig innerhalb des Gehäuses angeordnete Antenne der Funkübertragungseinrichtung ergibt sich ein einfacher Aufbau des Handgriffs ohne hervorstehende Teile. Dadurch ist das Handgriffgehäuse leichter zu reinigen und somit steril zu halten, was vor allem für den Einsatz im Operationssaal vorteilhaft ist.

Bei der Verwendung des Handgriffs an einer Operationsleuchte liegt die Antenne durch ihre Anordnung unterhalb der Operationsleuchte. Somit kann die Antenne eventuell vorhandene Reflexionskörper der Operationsleuchte, z.B. aus Aluminium, benutzen, um ihre Sendeleistung gerichtet in einen unteren Bereich eines Raumes abzustrahlen. Dies ist insbesondere von Vorteil, da ein Steuergerät bzw. eine darin vorgesehene Dekomprimierungseinrichtung im Operationssaal in der Regel in Tischhöhe angeordnet wird und sich so die Richtwirkung des Funksignals steigern lässt.

Die Funkübertragungseinrichtung kann als WLAN-Transceiver ausgebildet sein, der die HD-Videosignale insbesondere im WLAN-N Standard bei 5 GHz an die Dekomprimierungseinrichtung überträgt, die über verschiedene Video- und Audioausgänge für das HD-Videosignal, wie z.B. HD-SDi (High Definition Serial Digital Interface), DVI-D (Digital Visual Interface) oder HDMI (High Definition Multimedia Interface), verfügen kann.

Die Dekomprimierungseinrichtung in dem Steuergerät und die Funkübertragungseinrichtung in dem Handgriff können identische Platinen aufweisen, die automatisch erkennen ob sie Sender oder Empfänger sind und ob sie zur Komprimierung oder Dekomprimierung des HD-Videosignals eingesetzt werden sollen.

Vorteilhafte Ausführungsformen der Erfindung sind in der Beschreibung, den Zeichnungen sowie den Unteransprüchen beschrieben.

Nach einer ersten vorteilhaften Ausführungsform kann die Funkübertragungseinrichtung auch Steuersignale zur Einstellung der Operationsleuchte übermitteln, um z.B. die Helligkeit oder die Größe des ausgeleuchteten Feldes der Operationsleuchte anzupassen. Somit wird der ohnehin vorhandene Funkübertragungskanal doppelt genutzt, nämlich einerseits durch die Übertragung der Bildsignale und andererseits durch die Übermittlung der Steuersignale. Zugleich wird dadurch die Möglichkeit geschaffen, die Steuerleitungen für die Operationsleuchte in dem Halterungsgestänge wegzulassen, da die Steuersignale nun direkt von dem Handgriff zu der Operationsleuchte übertragen werden können.

Erfindungsgemäß kann nicht nur die Einstellung der Operationsleuchte über die übermittelten Steuersignale vorgenommen werden. Es sind auch weiterführende Einstellmöglichkeiten, wie z.B. die Steuerung der Raumbeleuchtung, das Starten einer Videoaufnahme oder die Verstellung des Operationstischs durch von dem Handgriff übermittelte Steuersignale möglich.

Nach einer weiteren vorteilhaften Ausführungsform umfasst der Handgriff lösbare Befestigungsmittel und eine Steckverbindung zur Energieversorgung. Dadurch wird der Handgriff austauschbar und kann an beliebigen geeigneten Operationsleuchten angebracht werden. Es ist denkbar, dass somit jeder Arzt bzw. Operateur immer seinen eigenen Handgriff benutzt, der für seine Bedürfnisse konfiguriert ist. Eine Konfiguration kann dabei u.a. die vom Operateur standardmäßig gewünschte Anfangshelligkeit der Beleuchtung und die Größe des ausgeleuchteten Bereichs umfassen. Ebenso kann die Konfiguration Einstellungen für die HD-Videokamera, wie z.B. das zu verwendende Video- oder Audioformat beinhalten.

Nach einer weiteren vorteilhaften Ausführungsform beinhaltet die Bildverarbeitungseinrichtung einen Computer, der einen Prozessor (z.B. einen ARM 9 Mikroprozessor), einen Arbeitsspeicher (RAM) und einen nichtflüchtigen Speicher (Flash-Speicher) aufweist und der mit einem eigenen autarken Betriebssystem, insbesondere Linux, versehen ist. Durch die Verwendung eines Computers zur Bildverarbeitung wird die Bildverarbeitung flexibel gestaltet. Es ist somit möglich spätere Änderungen z.B. des Videocodecs oder des Komprimierungsalgorithmus zu berücksichtigen.

Nach einer weiteren vorteilhaften Ausführungsform weist der Handgriff einen Griffabschnitt und einen Befestigungsabschnitt auf, wobei in dem Griffabschnitt die HD-Kamera und in dem Befestigungsabschnitt die übrigen elektronischen Komponenten, wie Bildverarbeitungseinrichtung, Komprimierungseinrichtung und Funkübertragungseinrichtung angeordnet sind. Im Befestigungsabschnitt können weiterhin Rändelschrauben, sowie elektrische Stecker angeordnet sein.

Die Rändelschrauben ermöglichen eine von Hand lösbare mechanische Verbindung mit der Operationsleuchte und die elektrischen Stecker stellen z.B. die Stromversorgung einer im Handgriff angeordneten Elektronik, insbesondere der Bildverarbeitungseinrichtung, sicher.

Nach einer weiteren vorteilhaften Ausführungsform kann der Griffabschnitt gegenüber dem Befestigungsabschnitt von Hand und zusätzlich durch einen Motor drehbar sein. Durch die Drehung des Griffabschnitts kann eine Einstellung der Operationsleuchte oder der HD-Kamera (camera control) vorgenommen werden. Es kann z.B. der ausgeleuchtete Bereich oder die Helligkeit der Operationsleuchte bzw. der Weißabgleich, der Zoom, die Blende oder die Rotation der HD-Kamera eingestellt werden.

Nach einer weiteren vorteilhaften Ausführungsform sind die elektronischen Komponenten der Bildverarbeitungseinrichtung, der Komprimierungseinrichtung und der Funkübertragungseinrichtung auf zwei übereinander geschichteten, insbesondere mehrlagigen Platinen angeordnet. Dadurch ergeben sich mehrere Vorteile. Zum einen können elektronische Komponenten, die sich z.B. durch hohe Oszillatorfrequenzen gegenseitig beeinflussen räumlich getrennt werden. Zum anderen erlaubt dies den Tausch einzelner Komponenten, z.B. bei einer Wartung oder bei einem Upgrade, leicht und kostengünstig durch die Auswechslung einzelner Platinen durchzuführen. Zusätzlich ergibt sich die Möglichkeit, wärmeempfindliche Bauteile bzw. Bauteile die eine starke Wärmeentwicklung besitzen auf derjenigen Platine anzuordnen, die im Betrieb einer geringeren Temperatur ausgesetzt ist bzw. die über eine bessere Wärmeableitung verfügt.

Der Einsatz von mehrlagigen Platinen ist auch vorteilhaft, um die EMV-Anforderungen an einen solchen Handgriff zu erfüllen. Da aufgrund der Übertragung der Videosignale über Antennen das (Kunststoff)-Gehäuse zumindest bereichsweise nicht geschirmt ist, muss die Abstrahlung von unerwünschter elektromagnetischer Strahlung bereits auf den Platinen unterbunden werden. Dies wird durch die oben genannte Verteilung der elektronischen Bauteile auf die Platinen sowie durch die Verwendung von mehrlagigen Platinen, die über einzelne Lagen zur Abschirmung elektromagnetischer Strahlung verfügen, erreicht.

Die verwendeten Platinen verfügen insbesondere über 16 Lagen, auf denen alle relevanten Leiterbahnen zur Verminderung der Abstrahlung elektromagnetischer Wellen gleich lang und mäanderförmig ausgeführt sein können.

Nach einer weiteren bevorzugten Ausführungsform sind auf der Oberseite einer oberen Platine ein DC-DC-Wandler und auf der Unterseite einer in Sandwichbauweise zu der oberen Platine angeordneten unteren Platine, ein Prozessor und ein Arbeitsspeicher sowie ein Videokomprimierungschip angeordnet. Somit ergeben sich kurze Leitungslängen, da die Bildverarbeitungseinrichtung nahe an der HD-Videokamera und die Stromversorgung (DC-DC-Wandler) nahe an den Anschlussleitungen an der Oberseite des Handgriffs angeordnet ist.

Die obere Platine kann weiterhin Bauteile aufweisen, die besonders wärmeempfindlich sind, da diese dort weit von den stark wärmeproduzierenden Bauteilen der unteren Platine entfernt sind. Aufgrund der auf der oberen Platine vorhandenen Verbindungsstecker zu der Operationsleuchte können dort auch Bauteile zur Implementierung eines CAN-Busses, zur Verstärkung, Verteilung oder A/D-Wandlung der Steuersignale sowie für ein USB-Interface angeordnet sein.

Somit kann die Operationsleuchte durch die Steuersignale, die z.B. über den CAN-Bus oder das USB-Interface von dem Handgriff übertragen werden können, gesteuert werden. Möglichkeiten sind u.a. die Steuerung der Leuchtfeldgröße, der Position des Reflektors oder der Ansteuerung einzelner LEDs der Operationsleuchte.

Nach einer weiteren vorteilhaften Ausführungsform dient ein Gehäuse der HD-Kamera als Kühlkörper. Dieses verfügt über eine große Oberfläche, die sich fast im gesamten Bereich des Griffabschnitts des Handgriffs erstreckt. Wegen der großen Oberfläche sind ein geringer Wärmewiderstand zwischen dem Gehäuse und der Umgebungsluft und somit eine gute Wärmeableitung gegeben.

Nach einer weiteren vorteilhaften Ausführungsform ist das Gehäuse der HD-Kamera mit den elektronischen Komponenten der Bildverarbeitungseinrichtung, insbesondere mit dem Prozessor, über ein Wärmeleitelement verbunden. Dies ist vorteilhaft, da in der Regel der Prozessor die größte Wärmequelle der Bildverarbeitungseinrichtung ist. Somit kann die Wärme der Bildverarbeitungseinrichtung über das Wärmeleitelement an das zur Kühlung vorgesehene Gehäuse der HD-Kamera übertragen werden.

Nachfolgend wird die vorliegende Erfindung rein beispielhaft anhand einer vorteilhaften Ausführungsform und unter Bezugnahme auf die Zeichnungen beschrieben.
- Fig. 1: zeigt einen schematischen Querschnitt durch einen Handgriff.
- Fig. 2: zeigt eine perspektivische Ansicht des Handgriffs mit einem Schnitt durch den Befestigungsabschnitt.

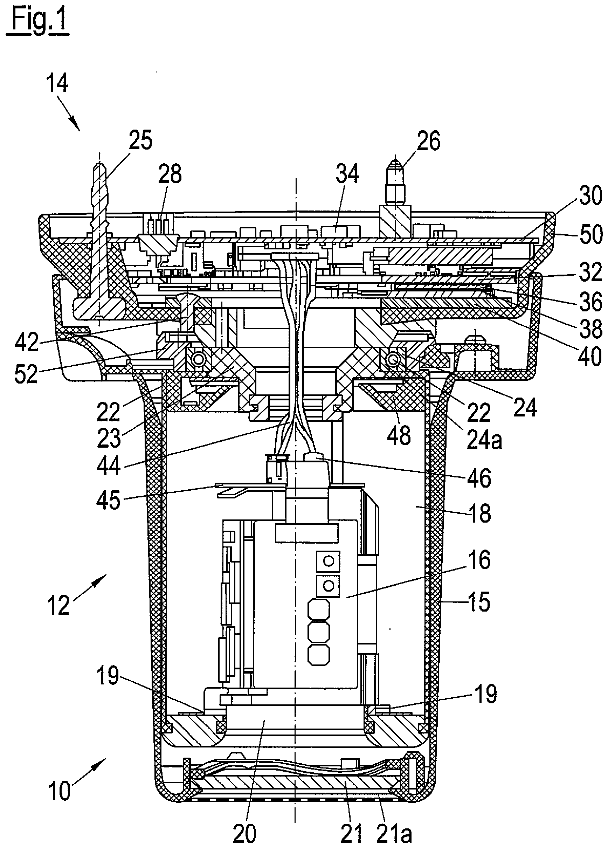

Der in Fig. 1 dargestellte Handgriff 10 für eine (nicht gezeigte) Operationsleuchte umfasst einen Griffabschnitt 12 und einen Befestigungsabschnitt 14. Der Griffabschnitt 12 ist unterhalb des Befestigungsabschnitts 14 angeordnet und wird außen im Wesentlichen von einer Griffhülse 15 bedeckt. Diese Griffhülse 15 ist auf ein Kameragehäuse 18 aufsteckbar und kann einzeln sterilisiert werden. Die Griffhülse 15 erhält ihre mechanisch korrekte Position durch das Kameragehäuse 18, auf das die Griffhülse 15 aufgeschoben ist.

Der Griffabschnitt 12 umfasst weiterhin in seinem Inneren eine HD-Kamera 16, die in dem Kameragehäuse 18 angeordnet ist. Die HD-Kamera 16 ist mittels einer Kamerahalterung 19 an dem Kameragehäuse 18 befestigt und besitzt eine nach unten gerichtete Sichtöffnung 20. Diese Sichtöffnung liegt oberhalb einer Klarglasscheibe 21, die ein auf der Unterseite der Griffhülse vorgesehenes Sichtfenster 21 a verschließt. Durch das Sichtfenster 21 a, die Klarglasscheibe 21 und die Sichtöffnung 20 ist der HD-Kamera 16 die Sicht nach unten freigegeben.

Der Griffabschnitt 12 weist an seinem oberen Ende ein Kugellager 22 auf durch das der Griffabschnitt 12 mit dem Befestigungsabschnitt 14 drehbar verbunden ist.

Hierzu ist ein innerer Ring 24a des Kugellagers 22 fest mit einer Antriebseinheit 23 verbunden, die über eine Kamerakopfaufnahme 48 eine feste Verbindung zwischen dem Kameragehäuse 18 und der Antriebseinheit 23 herstellt. Die Antriebseinheit 23 ist mit einem (nicht gezeigten) Getriebemotor versehen, der den Griffabschnitt 12 um die dargestellte vertikale Mittelachse gegenüber dem Befestigungsabschnitt 14 verdrehen kann. Somit ist neben der manuellen Drehung des Griffabschnitts 12 durch den Operateur auch eine automatische, von außen gesteuerte Drehung möglich. Durch die manuelle Drehung des Griffabschnitts 12 wird die Größe des von der Operationsleuchte beleuchteten Leuchtfelds eingestellt, wobei die Drehbewegung durch einen Drehgeber in Steuersignale umgewandelt wird.

Durch den Getriebemotor kann zudem eine von der Leuchtfeldeinstellung entkoppelte Rotation der Kamera vorgenommen werden, wobei die Steuerbefehle für den Getriebemotor über einen Funkkanal empfangen werden.

Ein äußerer Ring 24 des Kugellagers 22 ist über einen Verstärkungsring 52 mit einem nicht geschirmten oberen Gehäuse 50 verbunden, das drei Rändelschrauben 25 als lösbare Befestigungsmittel aufweist (wobei nur eine gezeigt ist), die den Handgriff 10 an der Operationsleuchte befestigen. Die Rändelschrauben 25 sind bei entfernter Griffhülse 15 zugänglich.

Die Rändelschrauben befestigt auch eine obere Platine 30, die diverse elektronische Bauelemente, unter anderem einen DC-DC-Wandler 34 zur Stromversorgung des Handgriffs 10 aufweist.

Auf der oberen Platine 30 sind weiterhin ein Büschelstecker 26 und ein Sub-D-Stecker 28 angeordnet, wobei der Büschelstecker 26 nach oben aus dem oberen Gehäuse 50 vorsteht. Die Stecker übertragen den Betriebsstrom für den Handgriff 10 sowie zusätzliche Steuersignale an die Operationsleuchte die über den Funkkanal empfangen werden.

Der Büschelstecker 26, durch den eine Masseverbindung hergestellt wird, steht dabei so weit nach oben aus dem oberen Gehäuse 50 heraus, dass bei einer Montage des Handgriffs 10 immer zuerst ein Masseanschluss erfolgt. Durch die Anordnung der Stecker 26 und 28 kann der Handgriff 10 nur in einer korrekten Orientierung an der Operationsleuchte montiert werden.

Eine zu der oberen Platine 30 parallele untere Platine 32 ist mittels Stehbolzen (nicht gezeigt) an der oberen Platine 30 befestigt. Die geschichteten und insbesondere mehrlagigen Platinen 30 und 32 sind zusätzlich über nicht gezeigte Steckverbinder elektrisch miteinander verbunden. Auf der unteren Platine sind ebenfalls elektronische Bauelemente für eine Bildverarbeitungseinrichtung, eine Komprimierungseinrichtung sowie eine Funkübertragungseinrichtung angeordnet.

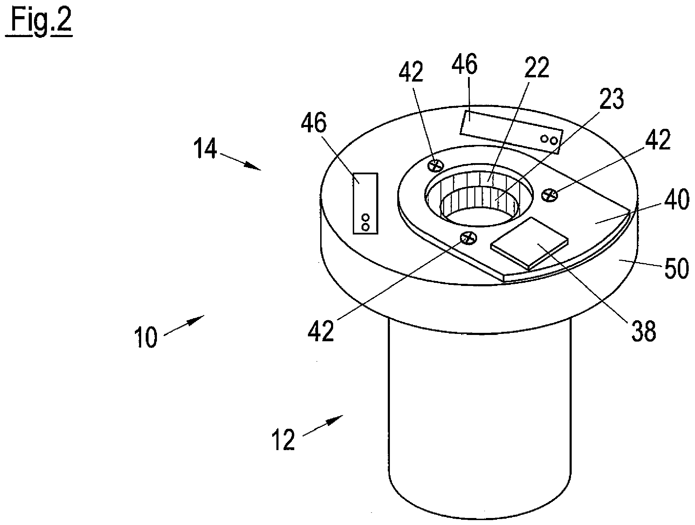

Neben der Funkübertragungseinrichtung sind Antennen 46 (vgl. Fig. 2) angeordnet, die Funksignale zu einer (nicht gezeigten) externen Steuerungseinheit übertragen. Die Funksignale können dabei Videosignale und Steuersignale zur Einstellung der Operationsleuchte und des Handgriffs 10 beinhalten.

Die Bildverarbeitungseinrichtung umfasst einen Computer, der einen Prozessor 36, einen Arbeitsspeicher und einen nicht flüchtigen Speicher aufweist, und der mit einem eigenen autarken Betriebssystem, insbesondere Linux, versehen ist.

Die Abwärme des Prozessors 36 wird über ein auf den Prozessor 36 aufgeklebtes Wärmeleitpad 38 zu einem Wärmeleitelement 40 übertragen (vgl. auch Fig. 2), wobei das Wärmeleitpad 38 auf der dem Prozessor abgewandten Seite mit dem Wärmeleitelement 40 verklebt ist. Beide Verklebungen des Wärmeleitpads 38 erfolgen mittels eines geeigneten Wärmeleitklebers.

Das Wärmeleitelement 40 ist grundsätzlich flächig ausgebildet und erstreckt sich im Wesentlichen in einer Ebene, die parallel zu den Platinen 30 und 32 liegt. Es gibt die ihm zugeführte Wärme im Wesentlichen über den Verstärkungsring 52, das Kugellager 22, die Antriebseinheit 23 und die Kamerakopfaufnahme 48 an das Kameragehäuse 18 ab. Über das Kameragehäuse 18 wird die am Prozessor entstandene Wärme an die Umgebungsluft abgeführt. Somit wird die große Oberfläche des Kameragehäuses 18 als Kühlkörper genutzt und führt die Wärme des Prozessors ab.

Die Verbindung der im Befestigungsabschnitt 14 angeordneten Platinen 30 und 32 mit der HD-Kamera 16 im Griffabschnitt 12 geschieht über elektrische Verbindungsleitungen 44. Diese sind zentral in dem Handgriff 10 angeordnet und werden so bei einer Drehung des Griffabschnitts 12 geringfügig verwunden, aber keiner Zugbelastung ausgesetzt. Die Verbindungsleitungen 44 weisen Datenübertragungsleitungen und Stromversorgungsleitungen auf, wobei auf der HD-Kamera 16 eine Adapterplatine 45 angeordnet ist, die über Adapterstecker 46 die HD-Kamera 16 mit den elektrischen Verbindungsleitungen 44 verbindet. Die HD-Kamera 16 ist so leicht durch Abstecken der Adapterstecker 46 auszutauschen.

Im Betrieb wird das von der HD-Kamera 16 erzeugte Videosignal durch die Bildverarbeitungseinrichtung komprimiert und mittels der Funkübertragungseinrichtung und der Antennen 46 an die externe Steuerungseinheit übertragen, die wiederum Steuersignale an die in dem Handgriff 10 vorgesehene Funkübertragungseinrichtung sendet. Auf Grundlage der Steuersignale wird die Einstellung der HD-Kamera 16 oder der Operationsleuchte vorgenommen, wobei die Steuersignale für die Operationsleuchte auf der oberen Platine 30 verstärkt und anschließend über den Sub-D-Stecker 28 übertragen werden.

Fig. 2 zeigt eine perspektivische Darstellung des Handgriffs 10 mit einem Schnitt durch den Befestigungsabschnitt. Dabei ist insbesondere die Lage des Wärmeleitelements 40 und des Wärmeleitpads 38 zu erkennen. Das Wärmeleitelement 40 ist durch Schrauben 42 an dem Kugellager 22 und der Antriebseinheit 23 befestigt. Die Antennen 46 sind im Befestigungsabschnitt 14 des Handgriffs 10 unterhalb der unteren Platine 32 angeordnet. Somit liegen die Antennen 46 nahe bei den Platinen 30 und 32 und benötigen keine unnötig langen und hochfrequenztechnisch ungünstigen Zuleitungen. Die Antennen 46 können insbesondere für den Betrieb bei 2,4 GHz oder 5 GHz ausgelegt sein.

### Bezugszeichenliste

- 10: Handgriff
- 12: Griffabschnitt
- 14: Befestigungsabschnitt
- 15: Griffhülse
- 16: HD-Kamera
- 18: Kameragehäuse
- 19: Kamerahalterung
- 20: Sichtöffnung
- 21: Klarglasscheibe
- 21a: Sichtfenster
- 22: Kugellager
- 23: Antriebseinheit
- 24: äußerer Ring
- 24a: innerer Ring
- 25: Rändelschrauben
- 26: Büschelstecker
- 28: Sub-D-Stecker
- 30: obere Platine
- 32: untere Platine
- 34: DC-DC-Wandler
- 36: Prozessor
- 38: Wärmeleitpad
- 40: Wärmeleitelement
- 42: Schrauben
- 44: Verbindungsleitungen
- 45: Adapterplatine
- 46: Adapterstecker
- 48: Kamerakopfaufnahme
- 50: oberes Gehäuse
- 52: Verstärkungsring

## Patentansprüche

1. Handgriff (10) für eine Operationsleuchte, in dessen Inneren eine HD-Kamera (16) angeordnet ist,
**dadurch gekennzeichnet, dass**
im Inneren des Handgriffs (10) weiterhin eine Bildverarbeitungseinrichtung, eine Komprimierungseinrichtung und eine Funkübertragungseinrichtung zur Übermittlung eines komprimierten HD-Videosignals und zumindest eine Antenne (46) vorgesehen sind, wobei die zumindest eine Antenne (46) vollständig innerhalb eines Gehäuses des Handgriffs angeordnet ist und das Gehäuse des Handgriffs (10) zumindest bereichsweise nicht geschirmt ist.

2. Handgriff (10) nach Anspruch 1,
**dadurch gekennzeichnet, dass** die Funkübertragungseinrichtung auch Steuersignale zur Einstellung der Operationsleuchte übermittelt.

3. Handgriff (10) nach zumindest einem der vorstehenden Ansprüche,
**gekennzeichnet durch**
lösbare Befestigungsmittel (25).

4. Handgriff (10) nach zumindest einem der vorstehenden Ansprüche,
**gekennzeichnet durch**
eine Steckverbindung (26, 28) zur Energieversorgung.

5. Handgriff (10) nach zumindest einem der vorstehenden Ansprüche,
**dadurch gekennzeichnet, dass**
die Bildverarbeitungseinrichtung einen Computer umfasst, der einen Prozessor (36), einen Arbeitsspeicher und einen nicht flüchtigen Speicher aufweist, und der mit einem eigenen autarken Betriebssystem versehen ist.

6. Handgriff (10) nach Anspruch 5,
**dadurch gekennzeichnet, dass** das Betriebssystem Linux ist.

7. Handgriff (10) nach zumindest einem der vorstehenden Ansprüche,
**gekennzeichnet durch**
einen Griffabschnitt (12), in welchem die HD-Kamera (16) angeordnet ist.

8. Handgriff (10) nach Anspruch 7,
**gekennzeichnet durch**
einen Befestigungsabschnitt (14), in welchem die übrigen elektronischen Komponenten wie Bildverarbeitungseinrichtung, Komprimierungseinrichtung und Funkübertragungseinrichtung angeordnet sind.

9. Handgriff (10) nach Anspruch 8,
**dadurch gekennzeichnet, dass**
der Griffabschnitt (12) gegenüber dem Befestigungsabschnitt (14) von Hand und durch einen Motor drehbar ist.

10. Handgriff (10) nach zumindest einem der vorstehenden Ansprüche,
**dadurch gekennzeichnet, dass** die elektronischen Komponenten der Bildverarbeitungseinrichtung, der Komprimierungseinrichtung und der Funkübertragungseinrichtung auf zwei übereinander geschichteten Platinen (30, 32) angeordnet sind.

11. Handgriff (10) nach Anspruch 10,
**dadurch gekennzeichnet, dass**
die Platinen (30, 32) mehrlagige Platinen (30, 32) sind.

12. Handgriff (10) nach Anspruch 10 oder 11,
**dadurch gekennzeichnet, dass**
auf der Oberseite der oberen Platine (30) ein DC-DC-Wandler (34) und auf der Unterseite der unteren Platine (32) ein Prozessor (36) und ein Arbeitsspeicher sowie eine Komprimierungseinrichtung angeordnet sind.

13. Handgriff (10) nach zumindest einem der vorstehenden Ansprüche,
**dadurch gekennzeichnet, dass**
ein Gehäuse (18) der HD-Kamera (16) als Kühlkörper dient.

14. Handgriff (10) nach Anspruch 13,
**dadurch gekennzeichnet, dass**
das Gehäuse (18) der HD-Kamera (16) mit zumindest einer elektronischen Komponente der Bildverarbeitungseinrichtung über ein Wärmeleitelement (40) verbunden ist.

15. Handgriff (10) nach Anspruch 14,
**dadurch gekennzeichnet, dass**
der Prozessor (36) über das Wärmeleitelement (40) mit dem Gehäuse (18) der HD-Kamera (16) verbunden ist.

## Claims

1. A handle (10) for a surgical light in whose interior an HD camera (16) is arranged,
**characterized in that**
furthermore an image processing device, a compression device and a radio transmission device for transmitting a compressed HD video signal and at least one antenna (46) are provided in the interior of the handle (10), wherein the at least one antenna (46) is arranged completely inside a housing of the handle and the housing of the handle (10) is not shielded at least regionally.

2. A handle (10) in accordance with claim 1,
**characterized in that**
the radio transmission device also transmits control signals for setting the surgical light.

3. A handle (10) in accordance with at least one of the preceding claims,
**characterized by**
releasable fastening means (25).

4. A handle (10) in accordance with at least one of the preceding claims,
**characterized by**
a plug-in connection (26, 28) for the energy supply.

5. A handle (10) in accordance with at least one of the preceding claims,
**characterized in that**
the image processing device comprises a computer which has a processor (36), a RAM memory and a non-volatile memory and which is provided with a separate autonomous operating system.

6. A handle (10) in accordance with claim 5,
**characterized in that**
the operating system is Linux.

7. A handle (10) in accordance with at least one of the preceding claims,
**characterized by**
a gripping section (12) in which the HD camera (16) is arranged.

8. A handle (10) in accordance with claim 7,
**characterized by**
a fastening section (14) in which the other electronic components such as the image processing device, the compression device and the radio transmission device are arranged.

9. A handle (10) in accordance with claim 8,
**characterized in that**
the gripping section (12) is rotatable by hand and by a motor with respect to the fastening section (14).

10. A handle (10) in accordance with at least one of the preceding claims,
**characterized in that**
electronic components of the image processing device, of the compression device and of the radio transmission device are arranged on two circuit boards (30, 32) layered above one another.

11. A handle (10) in accordance with claim 10,
**characterized in that**
the circuit boards (30, 32) are multilayer circuit boards (30, 32).

12. A handle (10) in accordance with claim 10 or claim 11,
**characterized in that**
a DC-DC converter (34) is arranged on the upper side of the upper circuit board (30) and a processor (36) and a RAM memory as well as a compression device are arranged on the lower side of the lower circuit board (32).

13. A handle (10) in accordance with at least one of the preceding claims,
**characterized in that**
a housing (18) of the HD camera (16) serves as a heat sink.

14. A handle (10) in accordance with claim 13,
**characterized in that**
the housing (18) of the HD camera (16) is connected to at least one electronic component of the image processing device via a thermally conductive element (40).

15. A handle (10) in accordance with claim 14,
**characterized in that**
the processor (36) is connected to the housing (18) of the HD camera (16) via the thermally conductive element (40).

## Revendications

1. Poignée (10) pour une lampe d'opération, à l'intérieur de laquelle est agencée une caméra haute définition (HD) (16),
**caractérisée en ce qu'**un système de traitement d'images, un système de compression et un système de transmission radio destiné à transmettre un signal vidéo HD comprimé et au moins une antenne (46) sont prévus en outre à l'intérieur de la poignée (10), et ladite au moins une antenne (46) est agencée entièrement à l'intérieur d'un boîtier de la poignée et le boîtier de la poignée (10) est au moins localement dépourvu de blindage.

2. Poignée (10) selon la revendication 1,
**caractérisée en ce que** le système de transmission radio transmet également des signaux de commande pour le réglage de la lampe d'opération.

3. Poignée (10) selon l'une au moins des revendications précédentes,
**caractérisée par** des moyens de fixation détachables (25).

4. Poignée (10) selon l'une au moins des revendications précédentes,
**caractérisée par** une connexion à enfichage (26, 28) pour l'alimentation en énergie.

5. Poignée (10) selon l'une au moins des revendications précédentes,
**caractérisée en ce que** le système de traitement d'images inclut un ordinateur qui comprend un processeur (36), une mémoire de travail et une mémoire non volatile, et qui est doté d'un système propre système d'exploitation autarcique.

6. Poignée (10) selon la revendication 5,
**caractérisée en ce que** le système d'exploitation est Linux.

7. Poignée (10) selon l'une au moins des revendications précédentes,
**caractérisée par** une portion de préhension (12) dans laquelle la caméra HD (16) est agencée.

8. Poignée (10) selon la revendication 7,
**caractérisée par** une portion de fixation (14) dans laquelle sont agencés les autres composants électroniques tels que le système de traitement d'images, le système de compression et le système de transmission radio.

9. Poignée (10) selon la revendication 8,
**caractérisée en ce que** la portion de préhension (12) est susceptible de tourner par rapport à la portion de fixation (12) à la main et au moyen d'un moteur.

10. Poignée (10) selon l'une au moins des revendications précédentes,
**caractérisée en ce que** les composants électroniques du système de traitement d'images, du système de compression et du système de transmission radio sont agencés sur de platine (30, 32) posées l'une au-dessus de l'autre.

11. Poignée (10) selon la revendication 10,
**caractérisée en ce que** les platines (30, 32) sont des platines à plusieurs couches (30, 32).

12. Poignée (10) selon la revendication 10 ou 11,
**caractérisée en ce qu'**un convertisseur continu/continu (34) est agencé sur la face supérieure de la platine supérieure (30), et **en ce qu'**un processeur (36) et une mémoire de travail ainsi qu'un système de compression sont agencés sur la face inférieure de la platine inférieure (32).

13. Poignée (10) selon l'une au moins des revendications précédentes,
**caractérisée en ce qu'**un boîtier (18) de la caméra HD (16) sert de corps de refroidissement.

14. Poignée (10) selon la revendication 13,
**caractérisée en ce que** le boîtier (18) de la caméra HD (16) est relié à au moins un composant électronique du système de traitement d'images via un élément thermoconducteur (40).

15. Poignée (10) selon la revendication 14,
**caractérisée en ce que** le processeur (36) est relié au boîtier (18) de la caméra HD (16) via l'élément thermoconducteur (40).
